# EUROPEAN PATENT SPECIFICATION

(11) **EP 4 453 239 B1**
(45) Date of publication and mention of the grant of the patent: **08.07.2026**
(21) Application number: 22840106.3
(22) Date of filing: 19.12.2022
(51) Int. Cl.: C12Q 1/6806

(54) **SURFACTANT FOR ANALYZING BIOLOGICAL MATERIAL**
TENSID ZUR ANALYSE VON BIOLOGISCHEM MATERIAL
TENSIOACTIF POUR L'ANALYSE D'UN MATÉRIAU BIOLOGIQUE

(30) Priority: 20.12.2021 EP 21306849
(43) Date of publication of application: 30.10.2024
(73) Proprietor: Stilla Technologies, 94800 Villejuif (FR)
(72) Inventor: URSUEGUI, Sylvain, 69005 Lyon (FR)
(74) Representative: Plasseraud IP
(86) International application number: PCT/EP2022/086592
(87) International publication number: WO 2023/117863

(56) References cited:
- WO-A1-2017/070363
- WO-A1-2020/116294
- WO-A2-2014/145582
- KANNER B ET AL: "SYNTHESIS AND PROPERTIES OF SILOXANE-POLYETHER COPOLYMER SURFACTANTS", INDUSTRIAL AND ENGINEERING CHEMISTRY PRODUCT RESEARCH AND DEVELOPMENT, AMERICAN CHEMICAL SOCIETY, EASTON, PA, US, vol. 6, no. 2, 1 June 1967 (1967-06-01), pages 88 - 92, XP000654117, ISSN: 0536-1079, DOI: 10.1021/I360022A002

## Description

### Field of the Invention

The invention pertains to the field of materials for analytical methods and relates to a composition for a microfluidic device.

### Background of the Invention

In the mid-1990s, advances in microfabrication techniques allowed the production of devices with feature sizes as small as few microns. Micromachining, photolithography, etching of silicon and glass substrates, all produced networks of flow channels that could sort reagents and molecules, partition chemical reactions, and act as droplet generators for mixture of aqueous and oil-based fluids. These microfluidic devices are well suited for performing biochemical assays, such as single-cell assays, studies of macromolecules e.g. proteins and nucleic acids, and polymerase chain reactions (PCR).

By the early 2000s, water-in-oil emulsions were being studied for potential applications designed to partition biological materials into nanoliter / picoliter-scale droplets. A significant advance was the use of surfactants in the oil-based continuous phase to maintain the integrity of droplets formed after the introduction of the discontinuous aqueous phase. Thanks to the use of surfactants in the oil-filled microfluidic devices, droplets can be pooled in widened channels or reservoirs, touching each other without breaking or fusing. Extensive fluidic networks of oil-and-surfactant filled channels can be designed to coordinate the intentional mixing of plugs or droplets to mediate biochemical reactions.

Fluorinated oils combined with fluorinated surfactants are particularly useful for handling biomolecules within aqueous droplets (Holtze et al., Biocompatible surfactants for water-in-fluorocarbon emulsions, 2008, 8(10), 1632-1639).

Emulsions containing fluorinated oil, however, can suffer from various disadvantages identified in the art. For example, aqueous droplets are typically buoyant in fluorinated oil (the density of fluorinated oil being higher than that of water), which may increase the complexity of droplet formation and manipulation: in some cases, the PCR mix deposition needs to be visually inspected; emulsions may require removal of excess oil below the droplets to position the droplets closer to a heat source, and/or the PCR mix position in the inlet well may be sensitive to inclination of the microfluidic chip.

Furthermore, the buoyant droplets may be more likely to be damaged by exposure to air above the emulsion, particularly when heated, the PCR mix is sensitive to evaporation leading to time constraints between sample deposition and sample analysis, and in some instances to the use of a sealing oil to prevent evaporation is necessary.

Hence, alternative systems have been proposed such as in WO2014/145582 which describes emulsions comprising aqueous droplets disposed in a continuous phase that includes a silicone oil and a silicone surfactant, wherein the silicone surfactant is described by the following general formula [SILICONE BACKBONE] [ALKYL]x[POLYETHER]y[POLYSILOXANE]z, where x is 0-5, y is 1-35, and z is 2-50.

WO2017070363 also discloses filler fluids for microfluidic devices comprising a silicone oil and a siloxane block co-polymer solubilized in the silicone oil, wherein the siloxane block co-polymer is substantially immiscible with an aqueous liquid.

However, the identification of a suitable surfactant/oil system, compatible with molecular biology, is a major aim for improving digital PCR performances.

The compatibility with molecular biology means that the system should not impair with the main reaction protocols used in molecular biology, such as PCR.

Digital PCR performances encompass, notably, droplets stability (meaning that the droplets are substantially prevented from mixing and merging when in contact with each other), increase of the number of droplets for a given volume of PCR mix, with the consequential increase of the analyzed volume, and finally, avoidance of buoyant droplets.

It is also a significant goal to simplify, when loading microfluidic chips, PCR mix deposition and positioning, to prevent PCR mix evaporation to avoid sample wasting, and finally to minimize dead volume, i.e. the volume of sample that cannot be analyzed.

### Summary of the Invention

The applicant identified a new category of surfactants and an oil/surfactant system, which are compatible with molecular biology, which improve digital PCR performances and limit sample wasting and dead volume.

The applicant indeed demonstrated that surfactants according to the invention prevent droplets from mixing and merging when in contact with each other and increase the droplets number and the analyzed volume compared to other surfactants.

This increase of droplets number and analyzed volume ensures the precision of digital PCR quantification and the separability of fluorescence signals between different populations, which are key parameters to determine robustness and precision of digital PCR.

Furthermore, the density ratio of the oil/surfactant system according to the invention is reversed compared to a fluorinated system, thus facilitating chip loading, preventing sample wasting and minimizing dead volume. These are major improvements for assay accuracy and reproducibility.

Finally, due to the density ratio inversion of a silicone oil compared to a fluorinated oil, the PCR mix is also protected against evaporation, thus allowing an extended operating time between chip loading and processing.

### Detailed Description

### Method for analyzing a biological material

In an embodiment, the present invention relates to a method for analyzing a biological material comprising:
- forming an emulsion which comprises said biological material, involving:
   - a continuous oily phase comprising a silicone oil;
   - a dispersed aqueous phase;
   - a surfactant which is a block copolymer comprising blocks [A], [B] and [C];
- processing said biological material in said emulsion; and
- detecting said biological material in said emulsion, wherein:
   - block [A] of the surfactant corresponds to a block of formula (I) 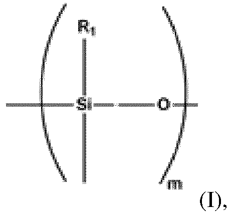 wherein R₁ is a C₁-C₁₈ alkyl, and m is comprised between 10 and 100;
   - block [B] of the surfactant corresponds to a block of formula (II) 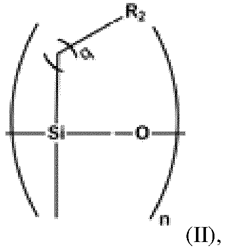 wherein q is comprised between 2 and 18, n is comprised between 1 and 50, and R₂ is selected from the group consisting of compounds of
      - 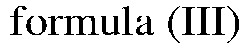 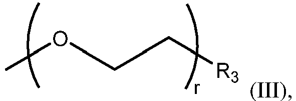 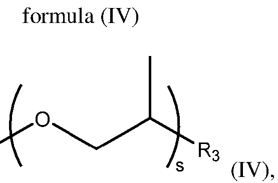
      - 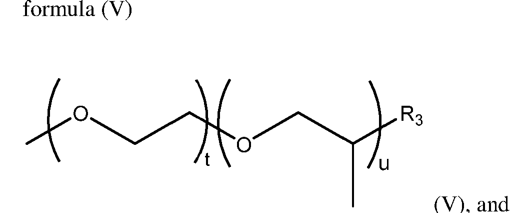 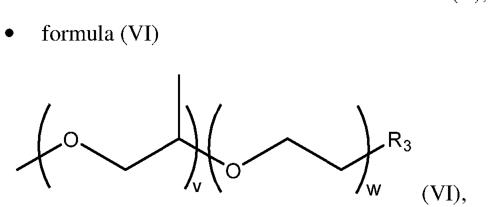 wherein r, s, t, u, v and w are comprised between 1 and 32 and R₃ is selected from the group consisting of -OH, -O-CH₃ and -O-CH₂CH₃;
   - block [C] of the surfactant corresponds to a block of formula (VII)

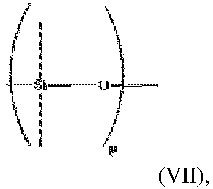

wherein p is comprised between 5 and 300;
wherein terminal groups are selected from the group consisting of -H, -CH₃ and -Si(CH₃)₃;
and wherein the general formula of the surfactant is selected from [A]-[B]-[C], [B]-[C]-[A], [C]-[A]-[B], [B]-[A]-[C], [A]-[C]-[B] and [C]-[B]-[A].

### Use of a silicone oil and a surfactant as an oily phase in digital PCR

In an embodiment, the present invention relates to the use of a silicone oil and a surfactant as an oily phase in digital PCR, wherein the surfactant is a block copolymer comprising blocks [A], [B] and [C], wherein:
- block [A] of the surfactant corresponds to a block of formula (I) 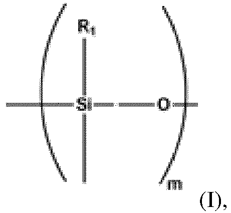 wherein R₁ is a C₁-C₁₈ alkyl, and m is comprised between 10 and 100;
- block [B] of the surfactant corresponds to a block of formula (II) 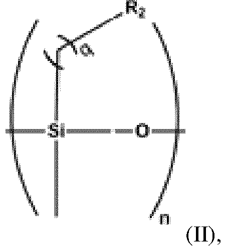 wherein q is comprised between 2 and 18, n is comprised between 1 and 50, and R₂ is selected from the group consisting of compounds of 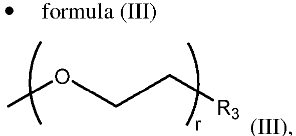 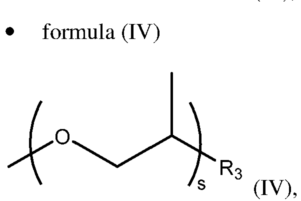
   - 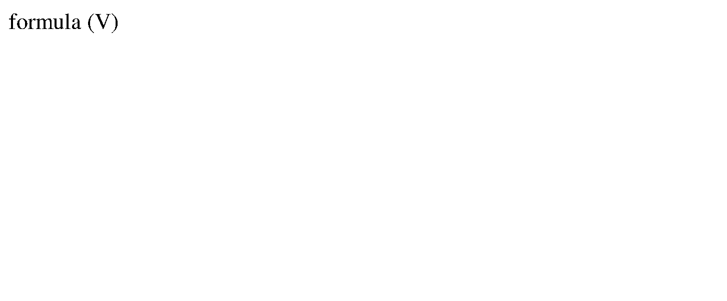 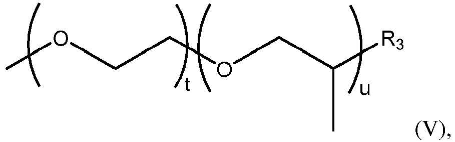 and 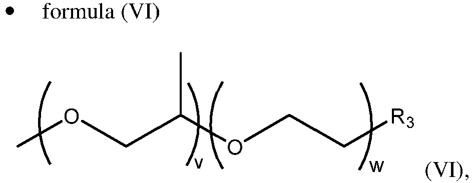
   wherein each of r, s, t, u, v and w are comprised between 1 and 32 and R₃ is selected from the group consisting of -OH, -O-CH₃ and -O-CH₂CH₃;
- block [C] of the surfactant corresponds to a block of formula (VII)

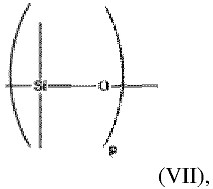

wherein p is comprised between 5 and 300;
wherein terminal groups are selected from the group consisting of -H, -CH₃ and -Si(CH₃)₃;
and wherein the general formula of the surfactant is selected from [A]-[B]-[C], [B]-[C]-[A], [C]-[A]-[B], [B]-[A]-[C], [A]-[C]-[B] and [C]-[B]-[A].

### Continuous oily phase for a microfluidic device

In an embodiment, the present invention relates to continuous oily phase for a microfluidic device comprising a silicone oil and a surfactant, wherein the surfactant is a block copolymer comprising blocks [A], [B] and [C], wherein:
- block [A] of the surfactant corresponds to a block of formula (I) 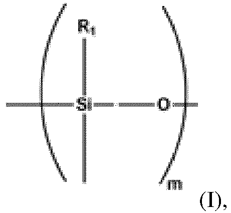 wherein R₁ is a C₁-C₁₈ alkyl, and m is comprised between 10 and 100;
- block [B] of the surfactant corresponds to a block of formula (II) 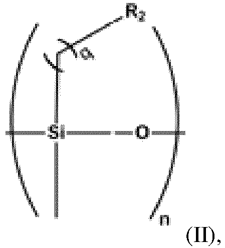 wherein q is comprised between 2 and 18, n is comprised between 1 and 50, and R₂ is selected from the group consisting of compounds of 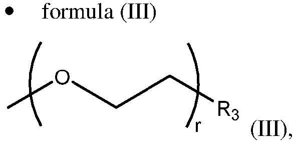 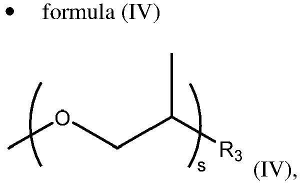
   - 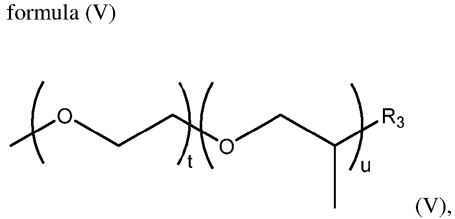 and 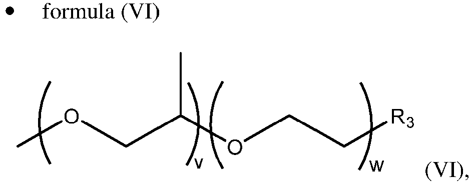 wherein each of r, s, t, u, v and w are comprised between 1 and 32 and R₃ is selected from the group consisting of -OH, -O-CH₃ and -O-CH₂CH₃;
- block [C] of the surfactant corresponds to a block of formula (VII)

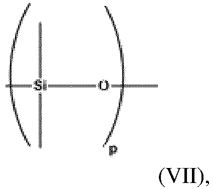

wherein p is comprised between 5 and 300;
wherein terminal groups are selected from the group consisting of -H, -CH₃ and -Si(CH₃)₃;
wherein the general formula of the surfactant is selected from [A]-[B]-[C], [B]-[C]-[A], [C]-[A]-[B], [B]-[A]-[C], [A]-[C]-[B] and [C]-[B]-[A];
and wherein the composition comprises at least 0,5 % by weight, relative to the total weight of the continuous oily phase, of the surfactant.

### Microfluidic device

In an embodiment, the present invention relates to a microfluidic device comprising at least one chamber containing a silicone oil and a surfactant, wherein the surfactant is a block copolymer comprising blocks [A], [B] and [C], wherein:
- block [A] of the surfactant corresponds to a block of formula (I) 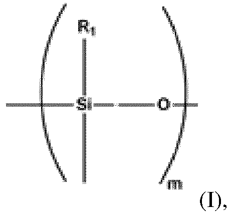 wherein R₁ is a C₁-C₁₈ alkyl, and m is comprised between 10 and 100;
- block [B] of the surfactant corresponds to a block of formula (II) 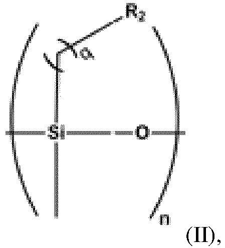 wherein q is comprised between 2 and 18, n is comprised between 1 and 50, and R₂ is selected from the group consisting of compounds of 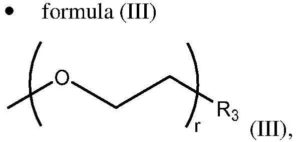 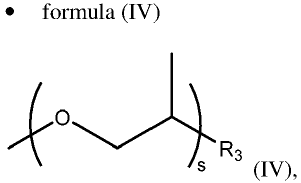
   - 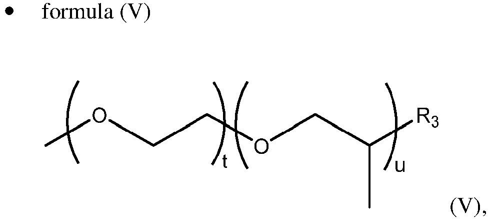 and 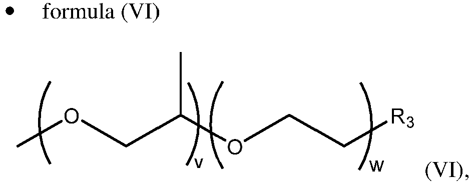 wherein each of r, s, t, u, v and w are comprised between 1 and 32 and R₃ is selected from the group consisting of -OH, -O-CH₃ and -O-CH₂CH₃;
- block [C] of the surfactant corresponds to a block of formula (VII)

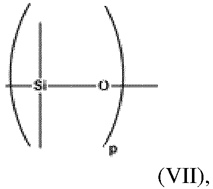

wherein p is comprised between 5 and 300;
wherein terminal groups are selected from the group consisting of -H, -CH₃ and -Si(CH₃)₃;
wherein the general formula of the surfactant is selected from [A]-[B]-[C], [B]-[C]-[A], [C]-[A]-[B], [B]-[A]-[C], [A]-[C]-[B] and [C]-[B]-[A].

### Surfactant

According to the invention, the surfactant is a block copolymer comprising blocks [A], [B] and [C].

In an embodiment, block [A] of the surfactant corresponds to a block of formula (I) 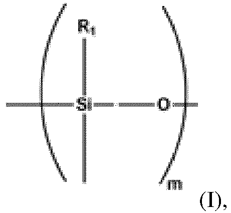 wherein R₁ is a C₁-C₁₈ alkyl, and m is comprised between 10 and 100.

In the above general formula, unless specified otherwise:
- Alkyl denotes a straight-chain or branched group containing 1, 2, 3, 4, 5, 6, 8, 9, 10, 11, 12, 13, 14, 15, 16, 16, 17, 18 carbon atoms. Examples of suitable alkyl radicals are methyl, ethyl, n-propyl, isopropyl, n-butyl, isobutyl, sec-butyl, tert-butyl, n-pentyl, isopentyl, n-hexyl, etc. Hence, a C₁-C₁₈ alkyl corresponds to an alkyl group containing 1 to 18 carbon atoms.

In an embodiment, m is comprised between 10 and 35, preferably between 10 and 17 or between 17 and 25.

In an embodiment, m is comprised between 25 and 75.

In an embodiment, R₁ is a C₁₂ alkyl.

In an embodiment, R₁ is a C₁₆ alkyl.

In an embodiment, R₁ is a C₁₂ alkyl and m is comprised between 10 and 35, preferably between 10 and 17 or between 17 and 25.

In an embodiment, R₁ is a C₁₆ alkyl and m is comprised between 25 and 75.

In an embodiment, block [B] of the surfactant corresponds to a block of formula (II) 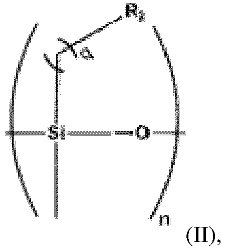 wherein q is comprised between 2 and 18, n is comprised between 1 and 50, and R₂ is selected from the group consisting of compounds of 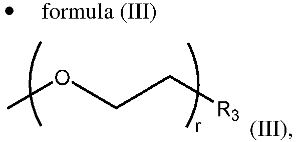 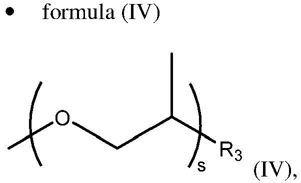
- 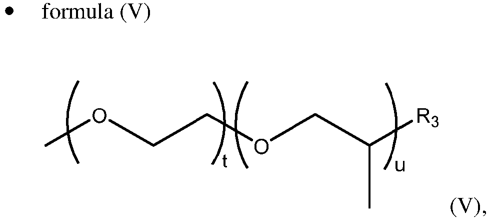 and 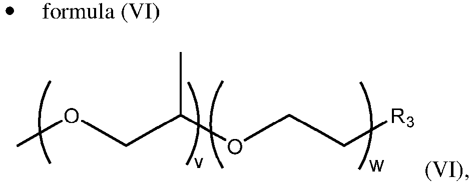
wherein r, s, t, u, v and w are comprised between 1 and 32 and R₃ is selected from the group consisting of -OH, -O-CH₃ and -O-CH₂CH₃.

In an embodiment, q is 3.

In an embodiment, n is comprised between 1 and 15, preferably 1 and 5.

In an embodiment, n is comprised between 5 and 15.

In an embodiment, r, s, t, u, v and w are comprised between 1 and 18, preferably between 1 and 12 and more preferably between 6 and 9.

In an embodiment, r, s, t, u, v and w are independently equal to 1 or 10.

In an embodiment, R₃ is -OH.

In an embodiment, R₂ is a compound of formula (III)

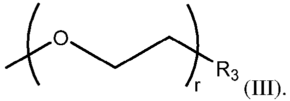

In an embodiment, R₂ is a compound of formula (V)

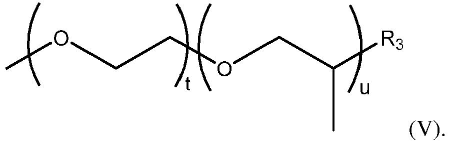

In an embodiment, R₂ is a compound of formula (III) 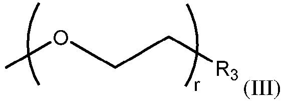 wherein r is comprised between 6 and 9 and R₃ is -OH.

In an embodiment, R₂ is a compound of formula (V) 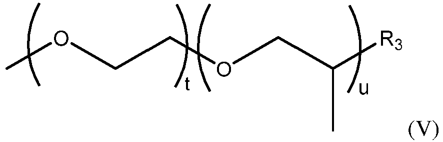 wherein t is 10, u is 1 and R₃ is -OH.

In an embodiment, block [C] of the surfactant corresponds to a block of formula (VII) 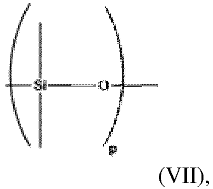 wherein p is comprised between 5 and 300.

In an embodiment, p is comprised between 5 and 200, preferably between 75 and 175 and more preferably between 95 and 140.

In an embodiment, p is comprised between 100 and 300.

In an embodiment, the block copolymer comprising blocks [A], [B] and [C] comprises terminal groups selected from the group consisting of -H, -CH₃ and -Si(CH₃)₃.

In an embodiment, the terminal groups are -CH₃ or -Si(CH₃)₃.

In an embodiment, the surfactant is a block polymer comprising blocks [A], [B] and [C], wherein
- block [A] of the surfactant corresponds to a block of formula (I) 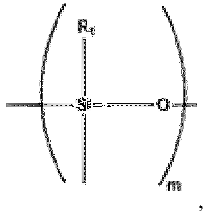 wherein R₁ is a C₁₂ alkyl and m is comprised between 10 and 35;
   - block [B] of the surfactant corresponds to a block of formula (II) 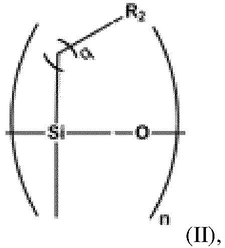 wherein q is 3, n is comprised between 1 and 15, and R₂ is a compound of formula (III) 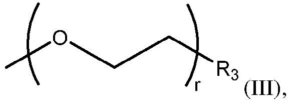 wherein r is comprised between 6 and 9 and R₃ is -OH;
   - block [C] of the surfactant corresponds to a block of formula (VII)

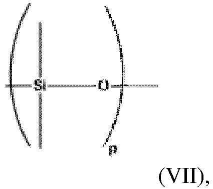

wherein p is comprised between 75 and 175;
wherein terminal groups are selected from the group consisting of H, CH₃ and -Si(CH₃)₃.

In an embodiment, the surfactant is a block polymer comprising blocks [A], [B] and [C], wherein
- block [A] of the surfactant corresponds to a block of formula (I) 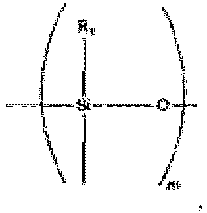 wherein R₁ is a C₁₆ alkyl and m is comprised between 25 and 75;
   - block [B] of the surfactant corresponds to a block of formula (II) 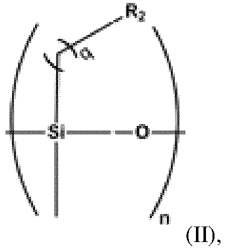 wherein q is 3, n is comprised between 1 and 5 or between 5 and 15, and R₂ is a compound of formula (V) 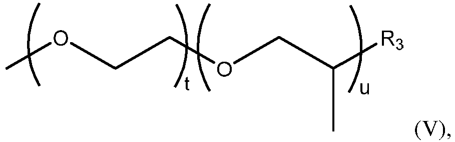 wherein u is 1, t is 10 and R₃ is -OH;
   - block [C] of the surfactant corresponds to a block of formula (VII)

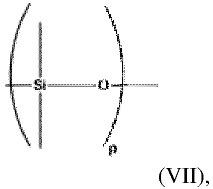

wherein p is comprised between 100 and 300;
and wherein terminal groups are selected from the group consisting of H, CH₃ and -Si(CH₃)₃.

In an embodiment, the general formula of the surfactant is [A]-[B]-[C].

In an embodiment, the general formula of the surfactant is [B]-[C]-[A].

In an embodiment, the general formula of the surfactant is [C]-[A]-[B].

In an embodiment, the general formula of the surfactant is [B]-[A]-[C].

In an embodiment, the general formula of the surfactant is [A]-[C]-[B].

In an embodiment, the general formula of the surfactant is [C]-[B]-[A].

According to an embodiment, surfactants that can be used in the system according to the invention can be selected from the group consisting of the surfactant commercialized by Gelest^{®} under the commercial name ABE 3642 (CAS No 212335-52-9), the surfactant commercialized by Siltech Corporation, under the commercial name Silube^{®}J208-812 (CAS No 212335-52-9) and the surfactant also commercialized by Siltech Corporation, under the commercial name Silube^{®} T310-A-16 (CAS No 145686-34-6).

In a preferred embodiment, the surfactant commercialized by Gelest^{®} under the commercial name ABE 3642 (CAS No 212335-52-9) can be used as a surfactant according to the present invention.

In a preferred embodiment, the surfactant comprises 15 to 50% by weight of the surfactant of block [A], preferably 18 to 50%, more preferably 20 to 40%.

In a preferred embodiment, the surfactant comprises 3 to 10% by weight of the surfactant of block [B].

In a preferred embodiment, the surfactant comprises 20 to 40% by weight of the surfactant of block [A], 3 to 10% by weight of the surfactant of block [B], and the remaining weight of the surfactant of block [C].

In an embodiment, the surfactant comprises 30 to 35% by weight of the surfactant of block [A], 7 to 10% by weight of the surfactant of block [B], and the remaining weight of the surfactant of block [C].

Typically, the percentages by weight are estimated by RMN 1H analysis.

### Continuous oily phase

According to the invention, the continuous oily phase comprises a silicone oil.

A silicone oil as used herein encompasses molecules having a skeleton structure of alternating silicon and oxygen atoms bonded one to another with hydrocarbon attached to silicon atoms. The silicon atoms may be substituted by various moieties, such as hydrocarbon moieties.

The silicon oil may be chosen according to its viscosity and optionally according to the design of the microfluidic device in which the continuous oily phase may be used.

Viscosity can be measured by any method known by the one skilled in the art. Typically, the viscosity can be measured using usual viscometer such as a rotational viscometer. Typically, the viscosimeter can be a viscosimeter adapted to low viscosity material. For example, Brookfield viscosimeters (AMETEK Brookfield, 11 Commerce Blvd., Middleboro, MA 02346 USA) such as LVT, LVDV-E, DV1MLV, DV2TLV, DV3LTV can be used, together with Brookfield low viscosity accessories.

In an embodiment, the continuous oily phase comprises a silicone oil with a viscosity between 0.5 and 500 centistokes at 25°C.

In an embodiment, the continuous oily phase comprises a silicone oil with a viscosity between 0.5 and 100 centistokes at 25°C.

In an embodiment, the continuous oily phase comprises a silicone oil with a viscosity between 0.5 and 50 centistokes at 25°C.

In an embodiment, the continuous oily phase comprises a silicone oil with a viscosity between 0.5 and 10 centistokes at 25°C.

In an embodiment, the continuous oily phase comprises a silicone oil with a viscosity between 0.7 and 5 centistokes at 25°C.

In an embodiment, the viscosity of the continuous oily phase is comprised between 0.8 and 4.0 centistokes at 25°C, preferably between 0.9 and 3.0 centistokes at 25°C, and more preferably between 1.0 and 2.0 centistokes at 25°C.

Typically, the viscosity of the continuous oily phase is between 1.1 and 1.5 centistokes at 25°C.

Silicone oils with a viscosity between 0.7 and 5 centistokes at 25°C are particularly advantageous when used in Opal^{™} microfluidic devices, described, for example, in patent applications WO2020/109379 and WO2020/109388.

In an embodiment, the silicone oil comprises decamethyltetrasiloxane.

In another embodiment, the silicone oil comprises at least 60% by weight relative to the total weight of the silicone oil of decamethyltetrasiloxane, preferably 70% by weight, relative to the total weight of the silicone oil of decamethyltetrasiloxane, more preferably 80% by weight relative to the total weight of the silicone oil of decamethyltetrasiloxane and even preferably 90% by weight relative to the total weight of the silicone oil of decamethyltetrasiloxane.

In another embodiment, the silicone oil further comprises octamethyltrisiloxane and/or dodecamethylpentasiloxane.

In an embodiment, the continuous oily phase comprises 1 to 10% by weight of octamethyltrisiloxane and/or 1 to 10% by weight of dodecamethylpentasiloxane.

In an embodiment, the product DMS-T01.5 (CAS No: 63148-62-9) commercialized by Gelest^{®} can be used as the silicone oil.

In an embodiment, the continuous oily phase comprises the surfactant according to the invention.

In an embodiment, the continuous oily phase comprises 0,5 to 5% by weight, relative to the total weight of the continuous oily phase, of the surfactant as previously defined, preferably, 0,5 to 2.5% by weight, relative to the total weight of the continuous oily phase, of the surfactant as previously defined, and more preferably, 0,5 to 1.5% by weight, relative to the total weight of the continuous oily phase, of the surfactant as previously defined.

In an embodiment, the continuous oily phase comprises about 1.0% by weight, relative to the total weight of the composition, of the surfactant as previously defined.

In a preferred embodiment, the continuous oily phase comprises a silicone oil comprising at least 60% of decamethyltetrasiloxane and a surfactant which is a block copolymer surfactant comprising blocks [A], [B] and [C], wherein
- block [A] of the surfactant corresponds to a block of formula (I) 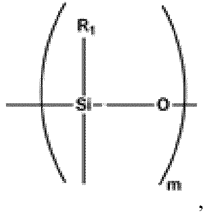 wherein R₁ is a C₁₂ alkyl and m is comprised between 10 and 35;
   - block [B] of the surfactant corresponds to a block of formula (II) 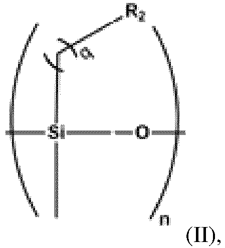 wherein q is 3, n is comprised between 1 and 15, and R₂ is a compound of formula (III) 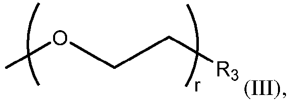 wherein r is comprised between 6 and 9 and R₃ is -OH;
   - block [C] of the surfactant corresponds to a block of formula (VII)

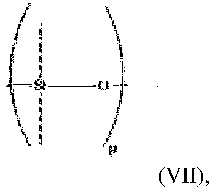

wherein p is comprised between 75 and 175;
and wherein terminal groups are selected from the group consisting of H, CH₃ and -Si(CH₃)₃.

In another preferred embodiment, the continuous oily phase comprises silicone oil comprising at least 60% of decamethyltetrasiloxane and a surfactant which is a block copolymer surfactant comprising blocks [A], [B] and [C], wherein
- block [A] of the surfactant corresponds to a block of formula (I) 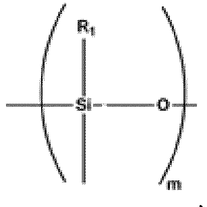 wherein R₁ is a C₁₆ alkyl and m is comprised between 25 and 75;
   - block [B] of the surfactant corresponds to a block of formula (II) 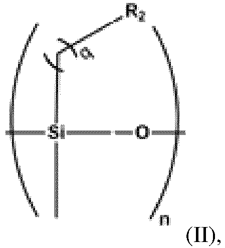 wherein q is 3, n is comprised between 1 and 5 or between 5 and 15, and R₂ is a compound of formula (V) 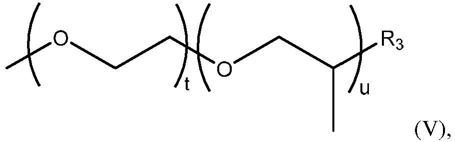 wherein u is 1, t is 10 and R₃ is -OH;
   - block [C] of the surfactant corresponds to a block of formula (VII)

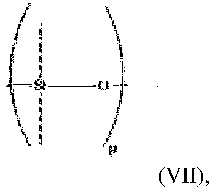

   wherein p is comprised between 100 and 300;
   and wherein terminal groups are selected from the group consisting of H, CH₃ and -Si(CH₃)₃.

### Dispersed aqueous phase

In an embodiment, the dispersed phase comprises water and any water miscible co-solvent, such as for example ethers glycol and polyether glycols, dimethyl sulfoxide (DMSO), short organic alcohols, acetone, short fatty acids, glycerol short organic amines, hydrogen peroxide, or organic and inorganic acids.

In an embodiment, the dispersed phase comprises reagents for performing a biological reaction and biological material.

### Biological material

Biological material refers, without limitation, to organisms, organs, tissues, cells (including eukaryotic and prokaryotic cells), viruses or virus particles, nucleic acids (including double stranded and single stranded DNA or RNA), plasmids, proteins, peptides, antibodies, enzymes, hormones, growth factors, carbohydrates and lipids, and derivatives, combinations, or polymers thereof. A "biological material" according to the present invention may be a material of natural or of synthetic origin. A "biological material" may be extracted, recovered, or obtained directly from a biological sample, such as, without limitation, blood, urine, cerebrospinal fluid, seminal fluid, saliva, sputum, stool, tissues or cells.

In a preferred embodiment, biological material refers to nucleic acids.

### Emulsion and population of droplets

"Emulsion" refers to a composition comprising at least two liquids, each of them being substantially immiscible in the other, wherein one of the liquids (which is referred to as the "dispersed phase") is partitioned into the other liquid (which is referred to as the "continuous phase"). The dispersed phase of an emulsion is typically suspended in the form of colloids, micelles, capsules and/or droplets. The dispersed phase and the continuous phase are typically fully immiscible. Emulsions are typically stabilized by inclusion in the emulsion of one or more surfactants and/or emulsifying agents.

In an embodiment, the present invention also relates to a population of droplets comprising an aqueous phase, dispersed in a continuous oily phase comprising a silicone oil and a surfactant.

All features detailed in the previous paragraphs entitled "dispersed aqueous phase", "continuous oily phase" and "surfactant" also apply to the emulsion and the population of droplets.

### Digital PCR

In an embodiment, the step of processing the biological material comprises amplifying the biological material, preferably by digital PCR, more preferably digital PCR in droplets.

Digital PCR may be seen to encompass three technologies, as follows:

### - Digital PCR in Microarrays:

With microfluidic-based digital PCR becoming well-known and more widely practiced, in 2006 Fluidigm Corporation commercialized the technology in an integrated microfluidic circuit. The BioMark system was based on the 1275 Digital Array, a chip of 12 panels, wherein each panel partitioned the reaction fluid into 765 6-nL chambers. After loading the PCR reaction mixture through 12 carrier inputs, the chip was thermocycled, fluorescence was detected, and the signal was processed and analysed by the Digital PCR Analysis software.

### - Digital PCR in Micro-droplets:

An alternative approach that further enhanced throughput and sensitivity while addressing the cost per reaction limitation was to generate picoliter-sized microdroplet reactors by flow-focusing offering many more partitions than the chamber-based systems. Droplets are generated in a microfluidic system, thermocycled to perform single-molecule digital PCR within the droplets, and end-point amplification is detected and quantified via real-time fluorescence curves. These droplet-based lab-on-chip systems were also adapted to perform reverse transcription PCR (RT-PCR) to detect single copies of RNA genomes and to perform multiplex reactions directed to multiple targets within a single droplet.

This technology was for example commercialized by QuantaLife, Inc. as the QX100 ddPCR^{™} System in 2011. The microfluidic consumables used on the ddPCR^{™} platform could accommodate up to eight samples per chip, generating 14,000-16,000 droplets per sample.

### - Crystal Digital PCR^{™}:

An advanced digital PCR equipment, the Naica^{®} System was launched in 2016 by Stilla Technologies. This system performs digital PCR by partitioning the sample, using a confinement gradient, into a large 2D array of droplets, also called a droplet crystal. A PCR reaction occurs in each of the partitioned 25-30,000 droplets that make up the droplet crystal, and a fluorescence read-out is performed at end point by taking high resolution image of the crystal.

Hence, and as used herein, the term "digital PCR" encompasses but is not limited to Digital PCR in Microarrays, Digital PCR in Micro-droplets and Crystal Digital PCR^{™}, and the term "digital PCR in droplets" encompasses Digital PCR in Micro-droplets and Crystal Digital PCR^{™}.

In an embodiment, the step of processing the biological material comprises amplifying the biological material, preferably by digital PCR and more preferably by digital PCR in droplets.

### Microfluidic device

The silicone oil/surfactant system according to the invention is advantageous in microfluidic devices comprising a droplet chamber.

As the density ratio of the oil/surfactant system according to the invention is reversed compared to that of a fluorinated system, chip loading is facilitated as the PCR mix sinks close to the droplet chamber, preventing sample wasting and minimizing dead volume.

The system according to the invention is particularly advantageous in Opal microfluidic devices, described, for example, in patent applications WO2020/109379 and WO2020/109388.

Opal microfluidic devices comprise at least one inlet microchannel, one droplet chamber, one output channel and a loading well configured to receive a drop of sample.

In the case of fluorinated system, the PCR mix floats in the loading well of the microfluidic chips. It may thus be necessary to inspect the mix deposition to ensure that the PCR mix is properly injected into the microfluidic chip. Furthermore, the mix position in the well is sensitive to inclination of the chip due to the floating nature of the droplets in the loading well.

On the contrary, in the case of the system according to the present invention, the PCR mix sinks into the loading well in proximity with the injector of the microfluidic chip. Hence, mix position is less sensitive to inclination and there is no need for inspection after mix pipetting. Chip loading is hence facilitated.

Furthermore, due to the density ratio inversion, the PCR mix is also protected against evaporation, allowing an extended operating time between chip loading and processing, and avoiding a supplementary step of dispensing sealing oil. This is also a major improvement for preventing sample wasting and minimizing dead volume.

The invention will be further illustrated by the following examples. However, these examples should not be interpreted in any way as limiting the scope of the present invention.

### Examples

### Material and Methods

### Protocol used in examples 1 and 2:

- Thaw the reagents - PCR Mix (Buffer A and B), Oligo Master Mix, H₂O and DNA stock.
- Thoroughly vortex and centrifuge each tube at least 10 sec.
- Prepare the following mix:
Use the following set up for 48 reactions of the 3-color run, using three Opal chips. Set up the PCR mix for all 48 samples in a batch mix to ensure equal conditions in each reaction.

| **Validation 3-plex assay** | | **Stock Solution** | **Final** | **volume (µL)** | **Pool** |
|---|---|---|---|---|---|
| | | **(µM or x)** | **(µM or x)** | **1** | **48** |
| Final volume expected | | | | 8 | 384 |
| Water | | | | 4.8 | 229.97 |
| naica multiplex PCR mix 5x (Buffer A) | | 5 | 1 | 1.6 | 76.8 |
| Valid. 3plex Oligos Pool (BRAF-FAM, pUC18 MSCL1-HEX, ALB-Cy5) | | 20 | 1 | 0.4 | 19.2 |
| Buffer B (100%) | | 100 | 4 | 0.32 | 15.36 |
| DNA pool {hgDNA + pUC18-LIN} | hgDNA cp/µL | 5400 | 600 | 0.89 | 42.67 |
| | pUC18 cp/µL | 81000 | 9000 | | |
| Final volume reached | | | | | 384.00 |

- Vortex and centrifuge the reaction mix.
- Use in Opal chips with 7 µL in each inlet port.
- Use in the naica^{®} Geode with the "Opal Template_PCR_45 cycles" program.
- Read chips on the Prism3 reader.
- Open data with Crystal Miner, check cluster separation in all three channels, as well as all the other criteria for assessing suitability of the tested surfactant/oil mixture for use in dPCR.

### Protocol used in example 3 - 3-plex assay

• Thaw the reagents - PCR Mix (Buffer A and B), Oligo Master Mix, H₂O and DNA stock.
• Thoroughly vortex and centrifuge each tube at least 10 sec.
• Prepare the following mix :
Use the following set up for 48 reactions of the 3-color run, using three Opal chips. Set up the PCR mix for all 48 samples in a batch mix to ensure equal conditions in each reaction.

| **Validation 3-plex assay** | | **Stock Solution** | **Final** | **volume (µL)** | **Pool** |
|---|---|---|---|---|---|
| | | **(µM or x)** | **(µM or x)** | **1** | **48** |
| Final volume expected | | | | 5.5 | 264.00 |
| Water | | | | 3.29 | 158.11 |
| naica multiplex PCR mix 5x (Buffer A) | | 5 | 1 | 1.1 | 52.8 |
| Valid. 3plex Oligos Pool (BRAF-FAM, pUC18 MSCL1-HEX, ALB-Cy5) | | 20 | 1 | 0.275 | 13.2 |
| Buffer B (100%) | | 100 | 4 | 0.22 | 10.56 |
| DNA pool {hgDNA + pUC18-LIN} | hgDNA cp/µL | 5400 | 600 | 0.61 | 29.33 |
| | pUC18 cp/µL | 81000 | 9000 | | |
| Final volume reached | | | | | 264.00 |

• Vortex and centrifuge the reaction mix.
• Use in Opal chips with 5 µL in each inlet port.
• Use in the naica^{®} Geode with a PCR 45 cycles program.
• Read chips on the Prism3 reader.
• Open data with Crystal Miner, check cluster separation in all three channels, as well as all the other criteria for assessing suitability of the tested surfactant/oil mixture for use in dPCR.
• Export quantification and separability data in a excel file.

### Protocol used in example 3 - 6-plex assay

• Thaw the reagents - PCR Mix (Buffer A and B), Oligo Master Mix, H₂O and DNA stock.
• Thoroughly vortex and centrifuge each tube at least 10 sec.
• Prepare the following mix :
Use the following set up for 48 reactions of the 6-color run, using three Opal chips. Set up the PCR mix for all 48 samples in a batch mix to ensure equal conditions in each reaction.

| **Validation 6-plex assay** | **WS** | **Final** | **volume (µL)** | **Pool** |
|---|---|---|---|---|
| | **(µM or x)** | **(µM or x)** | **1** | **48** |
| Final volume expected | | | 5.5 | 264 |
| Water | | | 3.02 | 144.91 |
| naica multiplex PCR mix 5x (Buffer A) | 5 | 1 | 1.10 | 52.80 |
| Valid. 6plex Primers Pool | 20 | 1 | 0.28 | 13.20 |
| Valid. 6plex Probes Pool | 20 | 1 | 0.28 | 13.20 |
| Buffer B (100%) | 100 | 4 | 0.22 | 10.56 |
| Valid. 6plex DNA pool | 27 | 3 | 0.61 | 29.33 |
| Final volume reached | | | 5.5 | 264.00 |

• Vortex and centrifuge the reaction mix.
• Use in Opal chips with 5 µL in each inlet port.
• Use in the naica^{®} Geode with a PCR 45 cycles program.
• Read chips on the Prism6 reader.
• Open data with Crystal Miner, check cluster separation in all three channels, as well as all the other criteria for assessing suitability of the tested surfactant for use in dPCR.
• Export quantification and separability data in an excel file.

### Results:

### Example 1: Droplet analysis

In a first set of PCR experiments, 8 different surfactants were used at a concentration of 5% w/w in decamethyltetrasiloxane.

Results obtained for 4 chambers (four replicates) per surfactant are described in the table below:

| **Surfactant** | **Droplet Analysis** | | |
|---|---|---|---|
| | **Droplet number (Mean)** | **Droplet number (CV %)** | **Analyzed volume µL (Mean)** |
| **AbEM 180** | 3760 | 63.26% | 1.23 |
| **AbEM 90** | 7003 | 42.86% | 2.06 |
| **ABE 3642** | 12361 | 17.81% | 2.86 |
| **DBE 224** | 5 | 146.24% | 0.00 |
| **AMS 163** | 5 | 200.00% | 0.00 |
| **Sigma 480320** | 0 | NA | 0.00 |
| **AbEM 97S** | 418 | 185.72% | 0.00 |
| **Silube J208-612** | 6 | 189.48% | 0.00 |

ABE 3642 advantageously increases the droplets number and the analyzed volume, compared to the other surfactants tested.In a second set of experiments, three new surfactants were screened, together with ABE 3642 as reference, at 1% w/w (as detailed below) in decamethyltetrasiloxane, following the same procedure previously used.

Results obtained for 16 chambers (sixteen replicates) per surfactant are described in the following table:

| **Surfactant (1 % w/w)** | **Droplet Analysis** | | |
|---|---|---|---|
| | **Droplet number (Mean)** | **Droplet number (CV %)** | **Analyzed volume µL (Mean)** |
| **ABE 3642** | 17190 | 12.04% | 3.67 |
| **Silube J208-812** | 8514 | 29.76% | 2.33 |
| **Silube T310-A16** | 3961 | 50.32% | 1.29 |
| **ABP 263** | 494 | 69.32% | 0.12 |
| **CMS 222** | 0 | ND | 0.00 |
| **CMS-222 0.1%** | 0 | ND | 0.00 |

The best results were obtained with ABE 3642 which enables to obtain the highest number of droplets and thus the highest analyzed volume. It is noteworthy that Silube J208-812 and Silube T310-A16 are also suitable to generate stable water-in-oil droplets while no stable droplets were observed with CMS-222 at 1% w/w or at 0.1% w/w.

CMS-222 is a siloxane block co-polymer represented by the following formula. This surfactant does not allow to obtain stable droplets.

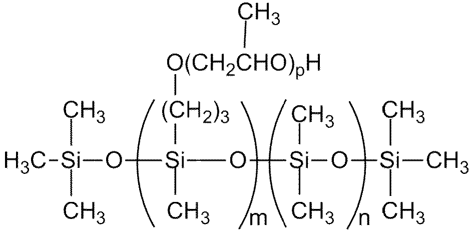

Without wishing to be bound by theory, it is hypothesized that polyethylene glycol chain of the [B] block within the surfactants according to the invention is a key parameter for preventing droplets from mixing and merging when in contact with each other and therefore a key parameter for increasing the droplets number and the analyzed volume compared to other surfactants. The alkyl chain of the [A] block within the surfactants according to the invention is potentially also a key parameter for preventing droplets from mixing and merging when in contact with each other and therefore a key parameter for increasing the droplets number and the analyzed volume compared to other surfactants.

Best results were obtained when the percentage by weight of surfactant of block [A] is in the range going from 20% to 40% by weight of surfactant and the percentage by weight of surfactant of block [B] is in the range going from 3% to 10% by weight of surfactant.

### Example 2: Surfactant concentration

The ABE 3642 surfactant was studied at a range of concentrations from 0.05% to 10% in decamethyltetrasiloxane in Opal chips.

| **ABE 3642 Concentration** | **Droplets Analysis** | | |
|---|---|---|---|
| | **Droplet number (Mean)** | **Droplet number (CV%)** | **Analyzed volume µL (Mean)** |
| **10%** | 7488 | 46.08% | 2.06 |
| **5%** | 8846 | 53.54% | 2.27 |
| **2.5%** | 13023 | 19.85% | 2.96 |
| **1%** | 10964 | 29.74% | 2.39 |
| **0.5%** | 12278 | 20.71% | 2.55 |
| **0.1%** | 5769 | 36.16% | 1.10 |

Stability of the 2D monolayer can be observed for surfactant concentrations of 0.5% w/w and above (as described in the table above), while concentrations above 5% w/w may be detrimental to biocompatibility (PCR could be inhibited by the too high concentration of surfactant).

### Example 3: Molecular biology compatibility experiments

In addition to the number of droplets, the precision of dPCR quantification, and the separability of fluorescence signals between different populations, were then assessed. All these experiments were performed with ABE 3642 at 1% (w/w) in decamethyltetrasiloxane.

The quantification accuracy was directly evaluated by comparison with results obtained with Opal chips filled with fluorinated chemistry.

For each channel, a satisfactory separability between negative and positive droplets was observed (separability index calculated by the Crystal Miner software of the naica^{®} Digital PCR suite > 4). No deviation of the quantification was observed compared to the reference concentration obtained with the chips filled with the fluorinated chemicals.

In addition to the 3-plex assay described above, a 6-plex assay was also performed to confirm suitability of the oil/surfactant mixture with other biological objects. This 6-plex assay uses Phage Lambda (blue channel), PhiX174 (teal channel), pUC18 MCS L1 (green channel), pBR 322 (yellow channel), Entero Cloaca (custom plasmid based on pUC57, red channel) and ALB (infra-red channel). The quantification accuracy was evaluated by comparison with target concentrations.

For all six channels, a satisfactory separability between negative and positive droplets was observed (separability > 4). No deviation of the quantification was observed compared to the target concentrations.

### Example 4: Evaluation of time of use between chip loading and chip processing

A comparative study was performed between the fluorinated system (Opal chips) and the silicon system (ABE 3642 1% in DMS-T01.5) to evaluate the effect of incubation time between chip loading and processing. For this, the chambers of each chip were loaded with the PCR mixture at 2-minute intervals and the chips were then processed in PCR such that the first chamber loaded had 26 minutes of incubation between chip loading and PCR while the last chamber loaded was processed immediately. Two chips per chemical system were processed with the 3-plex assay validation and the related protocol described for Example 3.

A clear increase in calculated concentration with the incubation time was observed in the fluorinated system, most likely due to evaporation of the PCR before injection, while calculated concentrations remained stable with the silicon system (i.e. an increase of 25% was observed in the green channel after 26 minutes for the fluorinated system while no deviation was observed with the silicon system).

In addition to the comparative studies with the fluorinated system, extended incubation times between chip loading and PCR have been evaluated with the silicon system (ABE 3642 1% in DMS-T01.5). The chips were loaded at the same time and then processed in dPCR after 6 hours or 10 hours of incubation at room temperature. The changes in calculated concentrations were evaluated by comparison to the results obtained when the chips are processed immediately after loading. The 3-plex validation assay and the related protocol described for Example 3 were also used for these tests.

Results obtained for 24 chambers. Chambers with less than 10K droplets were excluded before the analysis.

| **Use time** | **Blue channel** | | | **Green channel** | | | **Red channel** | | |
|---|---|---|---|---|---|---|---|---|---|
| | **Concentration cp/µL** | **Concentration CV** | **C/C(t0)** | **Concentration cp/µL** | **Concentration CV** | **C/C(t0)** | **Concentration cp/µL** | **Concentration CV** | **C/C(t0)** |
| t0 | 636.39 | 3.10% | NA | 9237.8 | 2.17% | NA | 651.26 | 2.95% | NA |
| t+6h | 677.49 | 3.51% | 106.5 % | 9566.82 | 3.34% | 103.6 % | 684.97 | 2.70% | 105.2 % |
| t+10h | 689.63 | 3.08% | 108.4 % | 9845.78 | 2.86% | 106.6 % | 708.92 | 2.85% | 108.9 % |

A small deviation in quantification of less than 10% for the three channels was observed after 6h and 10h of incubation at room temperature between chip loading and dPCR processing.

These results demonstrate that the silicon chemistry system according to the invention allows for an extended operating time between chip loading and processing, contrary to the fluorinated system of the state of the art.

## Claims

1. A method for analyzing a biological material comprising:
- forming an emulsion which comprises said biological material, involving:
- a continuous oily phase comprising a silicone oil;
- a dispersed aqueous phase;
- a surfactant which is a block copolymer comprising blocks [A], [B] and [C];
- processing said biological material in said emulsion; and
- detecting said biological material in said emulsion, wherein:
- block [A] of the surfactant corresponds to a block of formula (I) 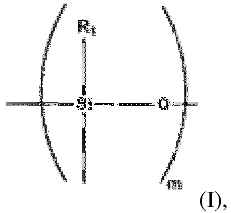 wherein R₁ is a C₁-C₁₈ alkyl, and m is comprised between 10 and 100;
- block [B] of the surfactant corresponds to a block of formula (II) 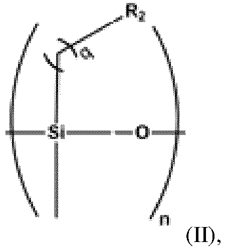 wherein q is comprised between 2 and 18, n is comprised between 1 and 50, and R₂ is selected from the group consisting of compounds of 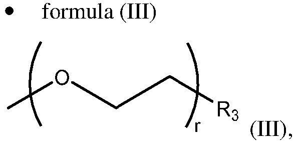
• 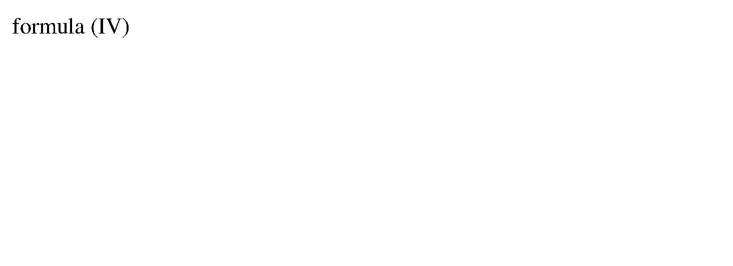 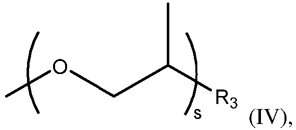
• 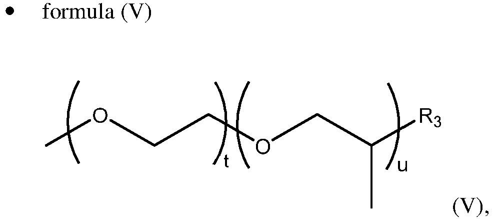 and 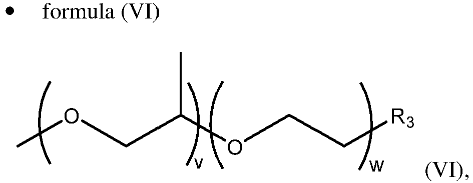 wherein r, s, t, u, v and w are comprised between 1 and 32 and R₃ is selected from the group consisting of -OH, -O-CH₃ and -O-CH₂CH₃;
- block [C] of the surfactant corresponds to a block of formula (VII) 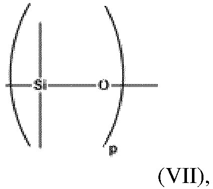
wherein p is comprised between 5 and 300;
wherein terminal groups are selected from the group consisting of -H, -CH₃ and -Si(CH₃)₃;
and wherein the general formula of the surfactant is selected from [A]-[B]-[C], [B]-[C]-[A], [C]-[A]-[B], [B]-[A]-[C], [A]-[C]-[B] and [C]-[B]-[A].

2. Use of a silicone oil and a surfactant as an oily phase in digital PCR, more preferably in digital PCR in droplets, wherein the surfactant is a block copolymer comprising blocks [A], [B] and [C], wherein:
- block [A] of the surfactant corresponds to a block of formula (I) 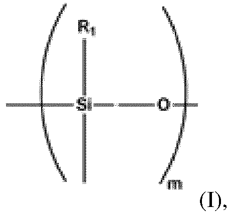 wherein R₁ is a C₁-C₁₈ alkyl, and m is comprised between 10 and 100;
- block [B] of the surfactant corresponds to a block of formula (II) 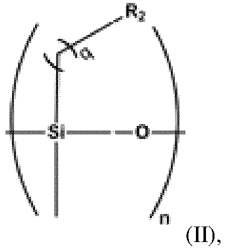 wherein q is comprised between 2 and 18, n is comprised between 1 and 50, and R₂ is selected from the group consisting of compounds of 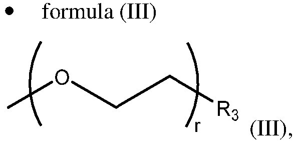 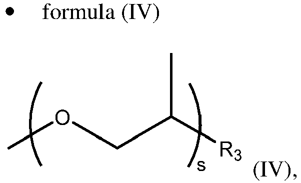
• 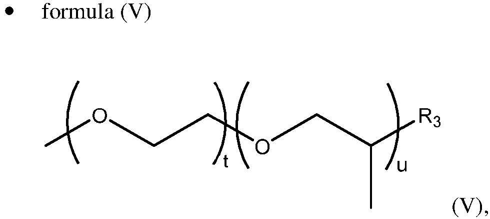 and
• 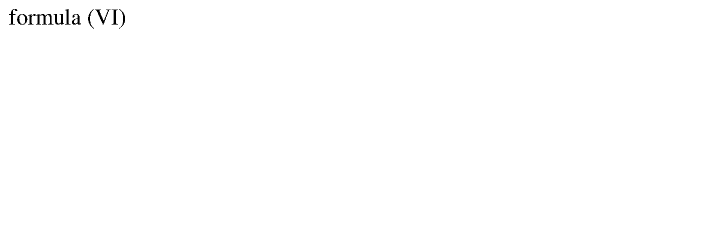 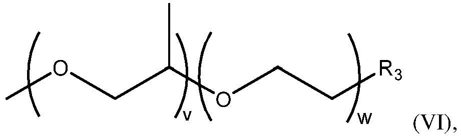 wherein each of r, s, t, u, v and w are comprised between 1 and 32 and R₃ is selected from the group consisting of -OH, -O-CH₃ and -O-CH₂CH₃;
- block [C] of the surfactant corresponds to a block of formula (VII) 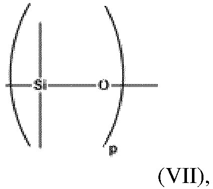
wherein p is comprised between 5 and 300;
wherein terminal groups are selected from the group consisting of -H, -CH₃ and -Si(CH₃)₃;
and wherein the general formula of the surfactant is selected from [A]-[B]-[C], [B]-[C]-[A], [C]-[A]-[B], [B]-[A]-[C], [A]-[C]-[B] and [C]-[B]-[A].

3. A continuous oily phase for a microfluidic device comprising a silicone oil and a surfactant, wherein the surfactant is a block copolymer comprising blocks [A], [B] and [C], wherein:
- block [A] of the surfactant corresponds to a block of formula (I) 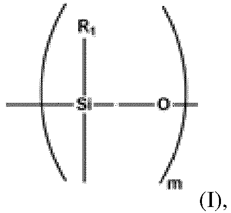 wherein R₁ is a C₁-C₁₈ alkyl, and m is comprised between 10 and 100;
- block [B] of the surfactant corresponds to a block of formula (II) 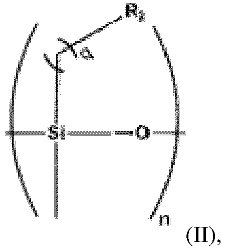 wherein q is comprised between 2 and 18, n is comprised between 1 and 50, and R₂ is selected from the group consisting of compounds of 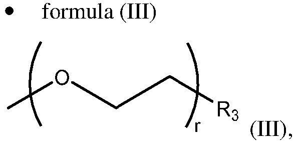 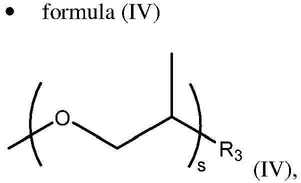
• 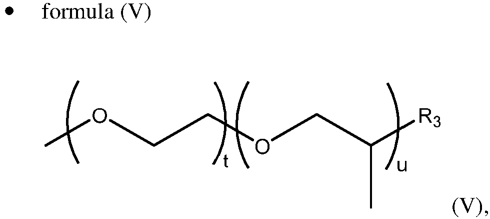 and 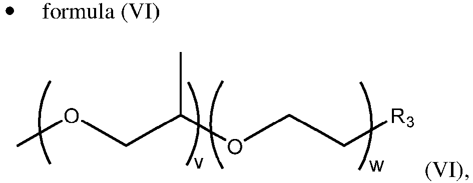 wherein each of r, s, t, u, v and w are comprised between 1 and 32 and R₃ is selected from the group consisting of -OH, -O-CH₃ and -O-CH₂CH₃;
- block [C] of the surfactant corresponds to a block of formula (VII) 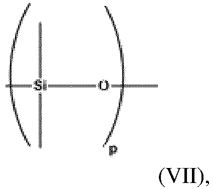
wherein p is comprised between 5 and 300;
wherein terminal groups are selected from the group consisting of -H, -CH₃ and -Si(CH₃)₃;
wherein the general formula of the surfactant is selected from [A]-[B]-[C], [B]-[C]-[A], [C]-[A]-[B], [B]-[A]-[C], [A]-[C]-[B] and [C]-[B]-[A];
and wherein the composition comprises at least 0,5 % by weight, relative to the total weight of the continuous oily phase, of the surfactant.

4. A microfluidic device comprising at least one chamber containing a silicone oil and a surfactant, wherein the surfactant is a block copolymer comprising blocks [A], [B] and [C], wherein:
- block [A] of the surfactant corresponds to a block of formula (I) 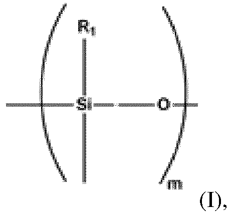 wherein R₁ is a C₁-C₁₈ alkyl, and m is comprised between 10 and 100;
- block [B] of the surfactant corresponds to a block of formula (II) 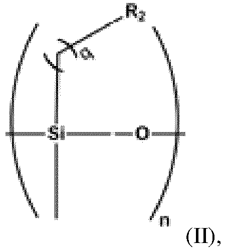 wherein q is comprised between 2 and 18, n is comprised between 1 and 50, and R₂ is selected from the group consisting of compounds of
• 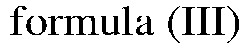 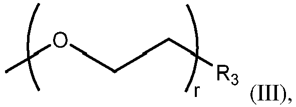 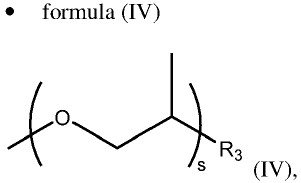
• 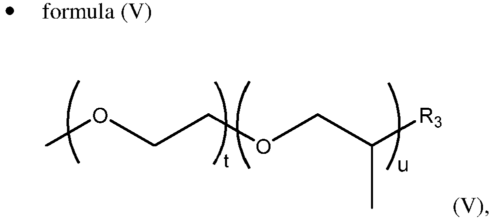 and 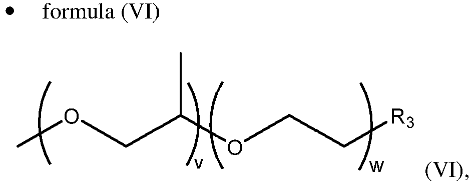 wherein each of r, s, t, u, v and w are comprised between 1 and 32 and R₃ is selected from the group consisting of -OH, -O-CH₃ and -O-CH₂CH₃;
- block [C] of the surfactant corresponds to a block of formula (VII) 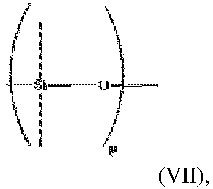
wherein p is comprised between 5 and 300;
wherein terminal groups are selected from the group consisting of -H, -CH₃ and -Si(CH₃)₃;
wherein the general formula of the surfactant is selected from [A]-[B]-[C], [B]-[C]-[A], [C]-[A]-[B], [B]-[A]-[C], [A]-[C]-[B] and [C]-[B]-[A].

5. The method according to claim 1, the use according to claim 2, the continuous oily phase according to claim 3 and the microfluidic device according to claim 4, wherein
- block [A] of the surfactant corresponds to a block of formula (I) 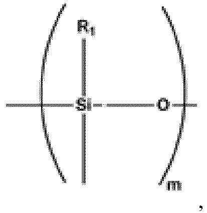 wherein R₁ is a C₁₂ alkyl and m is comprised between 10 and 35;
- block [B] of the surfactant corresponds to a block of formula (II) 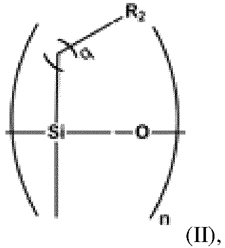 wherein q is 3, n is comprised between 1 and 15, and R₂ is a compound of formula (III) 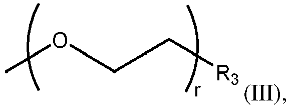 wherein r is comprised between 6 and 9 and R₃ is -OH;
- block [C] of the surfactant corresponds to a block of formula (VII) 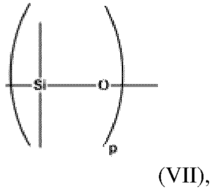
wherein p is comprised between 75 and 175;
wherein terminal groups are selected from the group consisting of H, CH₃ and -Si(CH₃)₃.

6. The method according to claim 1, the use according to claim 2, the continuous oily phase according to claim 3 and the microfluidic device according to claim 4, wherein
- block [A] of the surfactant corresponds to a block of formula (I) 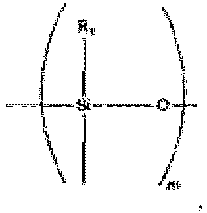 wherein R₁ is a C₁₆ alkyl and m is comprised between 25 and 75;
- block [B] of the surfactant corresponds to a block of formula (II) 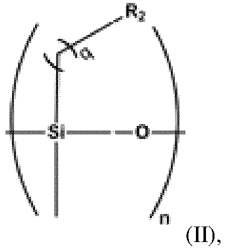 wherein q is 3, n is comprised between 1 and 5 or between 5 and 15, and R₂ is a compound of formula (V) 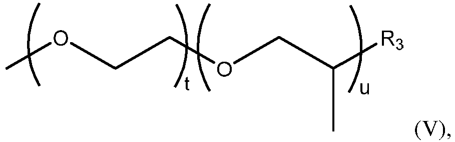 wherein u is 1, t is 10 and R₃ is -OH;
- block [C] of the surfactant corresponds to a block of formula (VII) 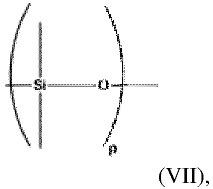
wherein p is comprised between 100 and 300;
wherein terminal groups are selected from the group consisting of H, CH₃ and -Si(CH₃)₃.

7. The method, the use, the continuous oily phase, the microfluidic device according to anyone of claims 1 to 6, wherein the viscosity of the silicone oil is comprised between 0.7 and 5.0 centistokes at 25°C.

8. The method, the use, the continuous oily phase, the microfluidic device according to anyone of claims 1 to 7, wherein the silicone oil comprises decamethyltetrasiloxane.

9. The method, the use, the continuous oily phase, the microfluidic device according to anyone of claims 1 to 8, wherein the silicone oil comprises at least 60% by weight of decamethyltetrasiloxane, relative to the total weight of the silicone oil.

10. The method, the use, the continuous oily phase, the microfluidic device according to anyone of claims 1 to 9, wherein the silicone oil further comprises octamethyltrisiloxane and/or dodecamethylpentasiloxane.

11. The method, the use, the continuous oily phase, the microfluidic device according to anyone of claims 1 to 10, wherein the continuous oily phase comprises 1 to 10% by weight of octamethyltrisiloxane and/or 1 to 10% by weight of dodecamethylpentasiloxane relative to the total weight of the silicone oil.

12. The method according to claim 1, wherein said biological material comprises cells or nucleic acids.

13. The method according to claim 1, wherein the step of processing the biological material comprises amplifying the biological material, preferably by digital PCR.

14. The method, the use, the continuous oily phase, the microfluidic device according to anyone of claims 1 to 13, wherein the surfactant is ABE 3642 being CAS No 212335-52-9

## Patentansprüche

1. Verfahren zum Analysieren eines biologischen Materials, umfassend:
- Bilden einer Emulsion, welche das biologische Material umfasst, beinhaltend:
- eine kontinuierliche ölige Phase, umfassend ein Silikonöl;
- eine dispergierte wässrige Phase;
- ein Tensid, welches ein Block-Copolymer ist, umfassend Blöcke [A], [B] und [C];
- Verarbeiten des biologischen Materials in der Emulsion; und
- Detektieren des biologischen Materials in der Emulsion, wobei:
- Block [A] des Tensids einem Block der Formel (I) entspricht 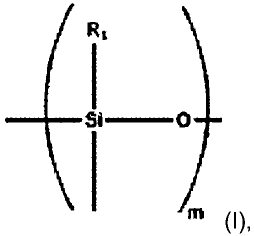 wobei R₁ ein C₁-C₁₈-Alkyl ist, und m zwischen 10 und 100 umfasst;
- Block [B] des Tensids einem Block der Formel (II) entspricht 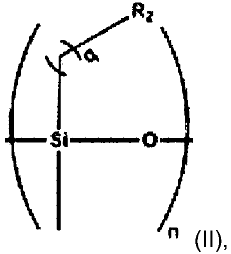 wobei q zwischen 2 und 18 umfasst, n zwischen 1 und 50 umfasst, und R₂ ausgewählt ist aus der Gruppe bestehend aus Verbindungen der 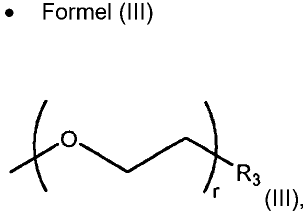 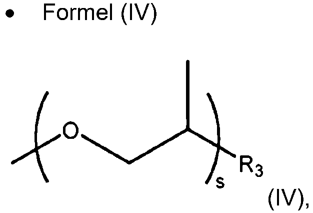 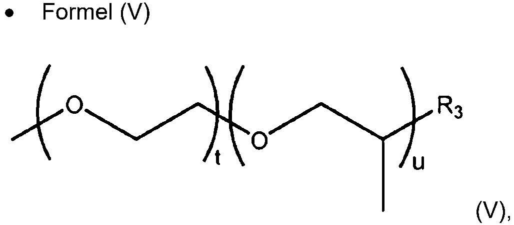 und 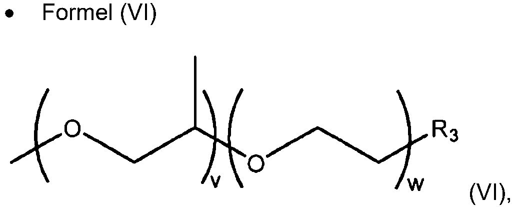 wobei r, s, t, u, v und w zwischen 1 und 32 umfassen und R₃ ausgewählt ist aus der Gruppe bestehend aus -OH, -O-CH₃ und -O-CH₂CH₃;
- Block [C] des Tensids einem Block der Formel (VII) entspricht 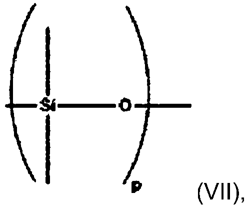
wobei p zwischen 5 und 300 umfasst;
wobei terminale Gruppen ausgewählt sind aus der Gruppe bestehend aus -H, -CH₃ und - Si(CH₃)₃;
und wobei die allgemeine Formel des Tensids ausgewählt ist aus [A]-[B]-[C], [B]-[C]-[A], [C]-[A]-[B], [B]-[A]-[C], [A]-[C]-[B] und [C]-[B]-[A].

2. Verwendung eines Silikonöls und eines Tensids als eine ölige Phase in digitaler PCR, stärker bevorzugt in digitaler PCR in Tröpfchen, wobei das Tensid ein Block-Copolymer ist, umfassend Blöcke [A], [B] und [C], wobei:
- Block [A] des Tensids einem Block der Formel (I) entspricht 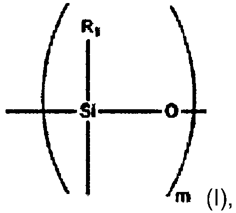 wobei R₁ ein C₁-C₁₈-Alkyl ist, und m zwischen 10 und 100 umfasst;
- Block [B] des Tensids einem Block der Formel (II) entspricht 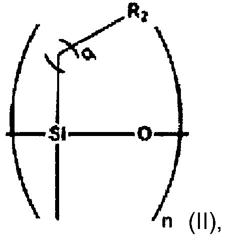 wobei q zwischen 2 und 18 umfasst, n zwischen 1 und 50 umfasst, und R₂ ausgewählt ist aus der Gruppe bestehend aus Verbindungen der 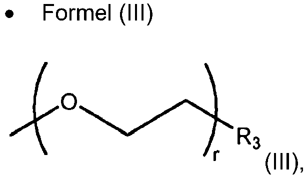 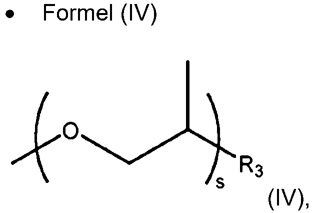 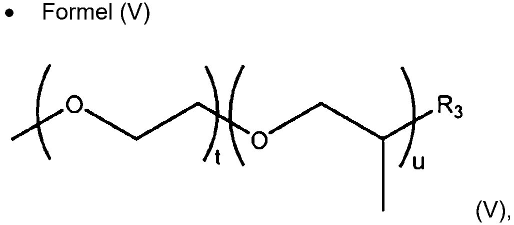 und 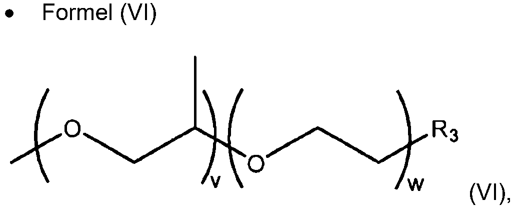 wobei jedes aus r, s, t, u, v und w zwischen 1 und 32 umfassen und R₃ ausgewählt ist aus der Gruppe bestehend aus -OH, -O-CH₃ und -O-CH₂CH₃;
- Block [C] des Tensids einem Block der Formel (VII) entspricht 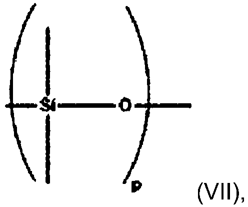
wobei p zwischen 5 und 300 umfasst;
wobei terminale Gruppen ausgewählt sind aus der Gruppe bestehend aus -H, -CH₃ und - Si(CH₃)₃;
und wobei die allgemeine Formel des Tensids ausgewählt ist aus [A]-[B]-[C], [B]-[C]-[A], [C]-[A]-[B], [B]-[A]-[C], [A]-[C]-[B] und [C]-[B]-[A].

3. Kontinuierliche ölige Phase für eine mikrofluidische Vorrichtung, umfassend ein Silikonöl und ein Tensid, wobei das Tensid ein Block-Copolymer ist, umfassend Blöcke [A], [B] und [C], wobei:
- Block [A] des Tensids einem Block der Formel (I) entspricht 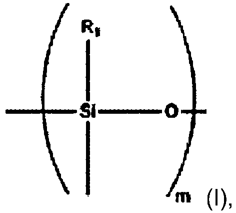 wobei R₁ ein C₁-C₁₈-Alkyl ist, und m zwischen 10 und 100 umfasst;
- Block [B] des Tensids einem Block der Formel (II) entspricht 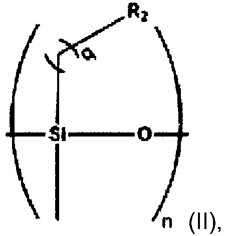 wobei q zwischen 2 und 18 umfasst, n zwischen 1 und 50 umfasst, und R₂ ausgewählt ist aus der Gruppe bestehend aus Verbindungen der 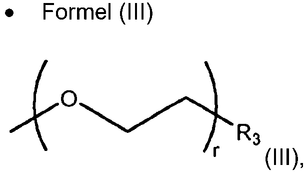 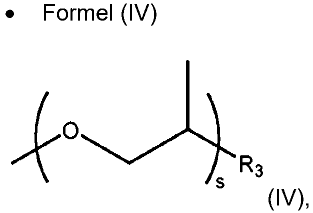 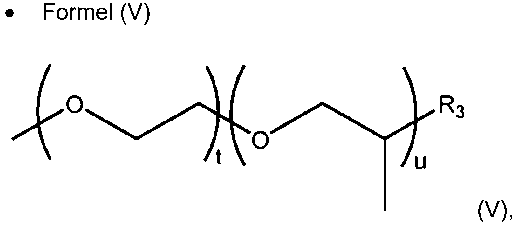 und 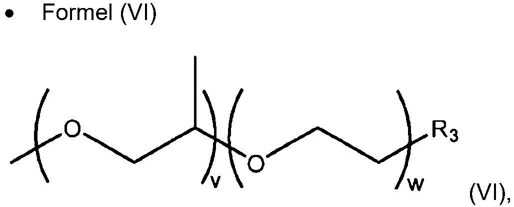 wobei jedes aus r, s, t, u, v und w zwischen 1 und 32 umfassen und R₃ ausgewählt ist aus der Gruppe bestehend aus -OH, -O-CH₃ und -O-CH₂CH₃;
- Block [C] des Tensids einem Block der Formel (VII) entspricht 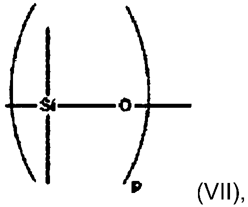
wobei p zwischen 5 und 300 umfasst;
wobei terminale Gruppen ausgewählt sind aus der Gruppe bestehend aus -H, -CH₃ und - Si(CH₃)₃;
wobei die allgemeine Formel des Tensids ausgewählt ist aus [A]-[B]-[C], [B]-[C]-[A], [C]-[A]-[B], [B]-[A]-[C], [A]-[C]-[B] und [C]-[B]-[A];
und wobei die Zusammensetzung mindestens 0,5 Gew.-% des Tensids, bezogen auf das Gesamtgewicht der kontinuierlichen öligen Phase, umfasst.

4. Mikrofluidische Vorrichtung, umfassend mindestens eine Kammer, welche ein Silikonöl und ein Tensid enthält, wobei das Tensid ein Block-Copolymer ist, umfassend Blöcke [A], [B] und [C], wobei:
- Block [A] des Tensids einem Block der Formel (I) entspricht 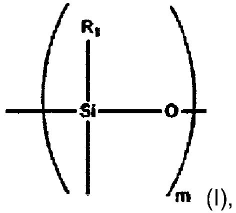 wobei R₁ ein C₁-C₁₈-Alkyl ist, und m zwischen 10 und 100 umfasst;
- Block [B] des Tensids einem Block der Formel (II) entspricht 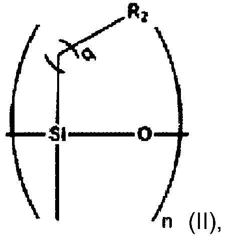 wobei q zwischen 2 und 18 umfasst, n zwischen 1 und 50 umfasst, und R₂ ausgewählt ist aus der Gruppe bestehend aus Verbindungen der 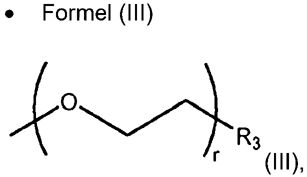 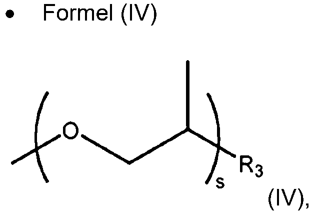 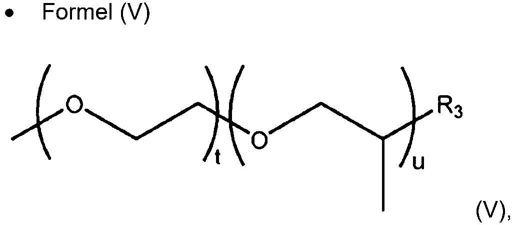 und 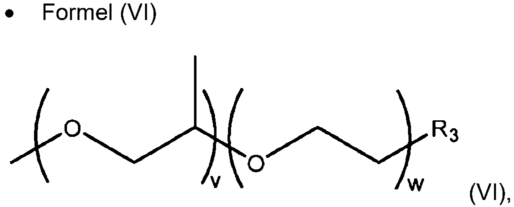 wobei jedes aus r, s, t, u, v und w zwischen 1 und 32 umfassen und R₃ ausgewählt ist aus der Gruppe bestehend aus -OH, -O-CH₃ und -O-CH₂CH₃;
- Block [C] des Tensids einem Block der Formel (VII) entspricht 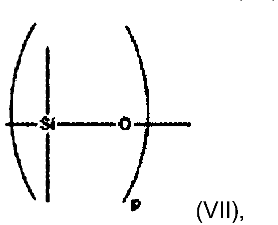 wobei p zwischen 5 und 300 umfasst;
wobei terminale Gruppen ausgewählt sind aus der Gruppe bestehend aus -H, -CH₃ und - Si(CH₃)₃;
wobei die allgemeine Formel des Tensids ausgewählt ist aus [A]-[B]-[C], [B]-[C]-[A], [C]-[A]-[B], [B]-[A]-[C], [A]-[C]-[B] und [C]-[B]-[A].

5. Verfahren nach Anspruch 1, Verwendung nach Anspruch 2, kontinuierliche ölige Phase nach Anspruch 3 und mikrofluidische Vorrichtung nach Anspruch 4, wobei
- Block [A] des Tensids einem Block der Formel (I) entspricht 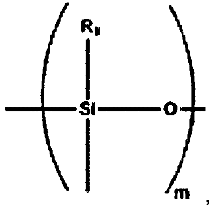 wobei R₁ ein C₁₂-Alkyl ist, und m zwischen 10 und 35 umfasst;
- Block [B] des Tensids einem Block der Formel (II) entspricht 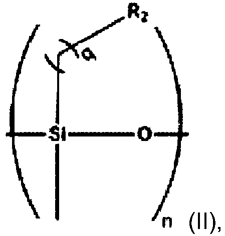 wobei q 3 ist, n zwischen 1 und 15 umfasst, und R₂ eine Verbindung der Formel (III) 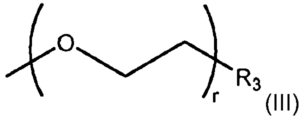 ist, wobei r zwischen 6 und 9 umfasst und R₃ -OH ist;
- Block [C] des Tensids einem Block der Formel (VII) entspricht 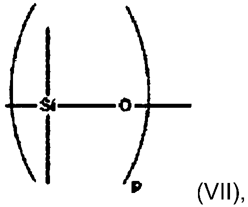
wobei p zwischen 75 und 175 umfasst;
wobei terminale Gruppen ausgewählt sind aus der Gruppe bestehend aus H, CH₃ und - Si(CH₃)₃.

6. Verfahren nach Anspruch 1, Verwendung nach Anspruch 2, kontinuierliche ölige Phase nach Anspruch 3 und mikrofluidische Vorrichtung nach Anspruch 4, wobei
- Block [A] des Tensids einem Block der Formel (I) entspricht 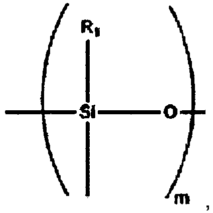 wobei R₁ ein C₁₆-Alkyl ist, und m zwischen 25 und 75 umfasst;
- Block [B] des Tensids einem Block der Formel (II) entspricht 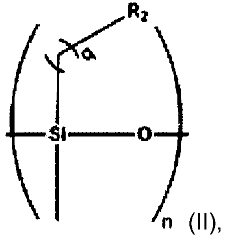 wobei q 3 ist, n zwischen 1 und 5 oder zwischen 5 und 15 umfasst, und R₂ eine Verbindung der Formel (V) 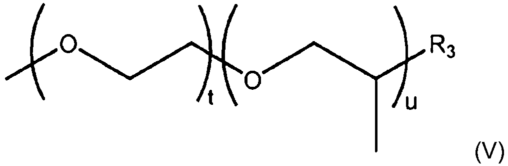 ist, wobei u 1 ist, t 10 ist und R₃ -OH ist;
- Block [C] des Tensids einem Block der Formel (VII) entspricht 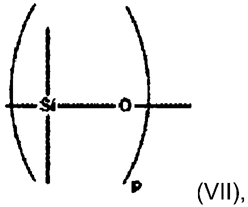
wobei p zwischen 100 und 300 umfasst;
wobei terminale Gruppen ausgewählt sind aus der Gruppe bestehend aus H, CH₃ und -Si(CH₃)₃.

7. Verfahren, Verwendung, kontinuierliche ölige Phase, mikrofluidische Vorrichtung nach einem der Ansprüche 1 bis 6, wobei die Viskosität des Silikonöls bei 25 °C zwischen 0,7 und 5,0 Centistokes liegt.

8. Verfahren, Verwendung, kontinuierliche ölige Phase, mikrofluidische Vorrichtung nach einem der Ansprüche 1 bis 7, wobei das Silikonöl Decamethyltetrasiloxan umfasst.

9. Verfahren, Verwendung, kontinuierliche ölige Phase, mikrofluidische Vorrichtung nach einem der Ansprüche 1 bis 8, wobei das Silikonöl mindestens 60 Gew.-% Decamethyltetrasiloxan, bezogen auf das Gesamtgewicht des Silikonöls, umfasst.

10. Verfahren, Verwendung, kontinuierliche ölige Phase, mikrofluidische Vorrichtung nach einem der Ansprüche 1 bis 9, wobei das Silikonöl ferner Octamethyltrisiloxan und/oder Dodecamethylpentasiloxan umfasst.

11. Verfahren, Verwendung, kontinuierliche ölige Phase, mikrofluidische Vorrichtung nach einem der Ansprüche 1 bis 10, wobei die kontinuierliche ölige Phase 1 bis 10 Gew.-% Octamethyltrisiloxan und/oder 1 bis 10 Gew.-% Dodecamethylpentasiloxan, bezogen auf das Gesamtgewicht des Silikonöls, umfasst.

12. Verfahren nach Anspruch 1, wobei das biologische Material Zellen oder Nukleinsäuren umfasst.

13. Verfahren nach Anspruch 1, wobei der Schritt des Verarbeitens des biologischen Materials Amplifizieren des biologischen Materials umfasst, bevorzugt mittels digitaler PCR.

14. Verfahren, Verwendung, kontinuierliche ölige Phase, mikrofluidische Vorrichtung nach einem der Ansprüche 1 bis 13, wobei das Tensid ABE 3642 ist, mit CAS-Nr. 212335-52-9.

## Revendications

1. Procédé d'analyse d'un matériel biologique, comprenant :
- la formation d'une émulsion qui comprend ledit matériel biologique, comportant :
- une phase huileuse continue comprenant une huile de silicone ;
- une phase aqueuse dispersée ;
- un tensioactif qui est un copolymère à blocs comprenant les blocs [A], [B] et [C] ;
- le traitement dudit matériel biologique dans ladite émulsion ; et
- la détection dudit matériel biologique dans ladite émulsion, dans lequel :
- le bloc [A] du tensioactif correspond à un bloc de formule (I) 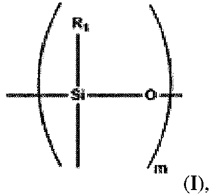 où R₁ est un alkyle en C₁-C₁₈ et m est compris entre 10 et 100 ;
- le bloc [B] du tensioactif correspond à un bloc de formule (II) 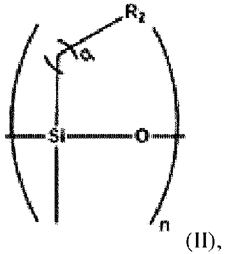 où q est compris entre 2 et 18, n est compris entre 1 et 50 et R₂ est choisi parmi le groupe constitué de composés de 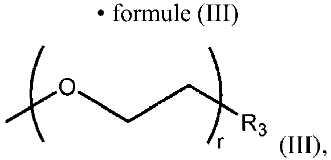
• 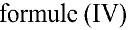 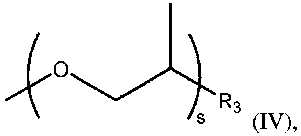 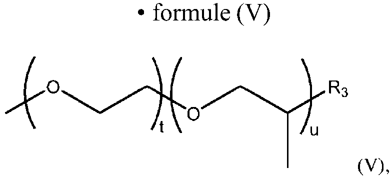 and 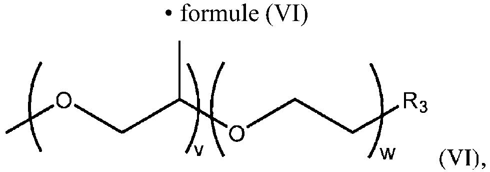 où r, s, t, u, v et w sont compris entre 1 et 32 et R₃ est choisi parmi le groupe constitué de -OH, -O-CH₃ et -O-CH₂CH₃ ;
- le bloc [C] du tensioactif correspond à un bloc de formule (VII) 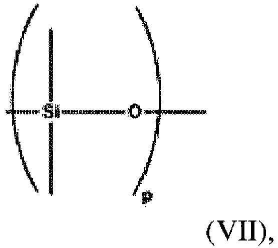
où p est compris entre 5 et 300 ;
où les groupes terminaux sont choisis parmi le groupe constitué de -H, -CH₃ et - Si(CH₃)₃ ;
et où la formule générale du tensioactif est choisie parmi [A]-[B]-[C], [B]-[C]-[A], [C]-[A]-[B], [B]-[A]-[C], [A]-[C]-[B] et [C]-[B]-[A].

2. Utilisation d'une huile de silicone et d'un tensioactif en tant que phase huileuse dans la PCR numérique, de préférence dans la PCR numérique en gouttelettes, dans laquelle le tensioactif est un copolymère à blocs comprenant des blocs [A], [B] et [C], dans laquelle :
- le bloc [A] du tensioactif correspond à un bloc de formule (I) 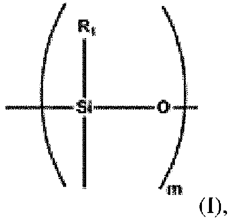 où R₁ est un alkyle en C₁-C₁₈ et m est compris entre 10 et 100 ;
- le bloc [B] du tensioactif correspond à un bloc de formule (II) 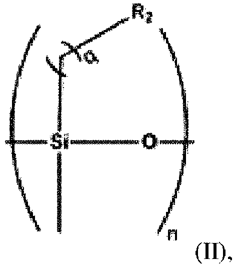 où q est compris entre 2 et 18, n est compris entre 1 et 50 et R₂ est choisi parmi le groupe constitué de composés de 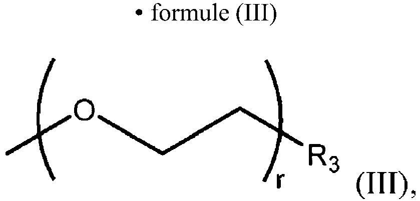 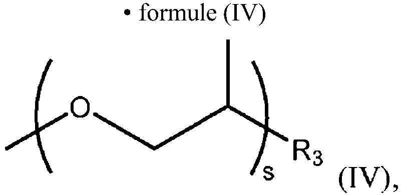 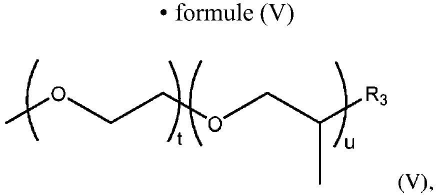 and 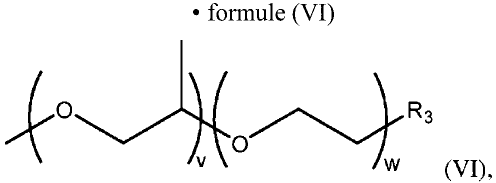 où chacun de r, s, t, u, v et w est compris entre 1 et 32 et R₃ est choisi parmi le groupe constitué de -OH, -O-CH₃ et -O-CH₂CH₃ ;
- le bloc [C] du tensioactif correspond à un bloc de formule (VII) 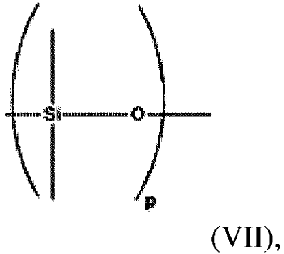
où p est compris entre 5 et 300 ;
où les groupes terminaux sont choisis parmi le groupe constitué de -H, -CH₃ et - Si(CH₃)₃ ;
et où la formule générale du tensioactif est choisie parmi [A]-[B]-[C], [B]-[C]-[A], [C]-[A]-[B], [B]-[A]-[C], [A]-[C]-[B] et [C]-[B]-[A].

3. Phase huileuse continue pour un dispositif microfluidique comprenant une huile de silicone et un tensioactif, dans laquelle le tensioactif est un copolymère à blocs comprenant les blocs [A], [B] et [C], dans laquelle :
- le bloc [A] du tensioactif correspond à un bloc de formule (I) 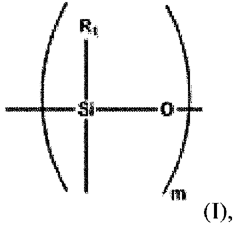 où R₁ est un alkyle en C₁-C₁₈ et m est compris entre 10 et 100 ;
- le bloc [B] du tensioactif correspond à un bloc de formule (II) 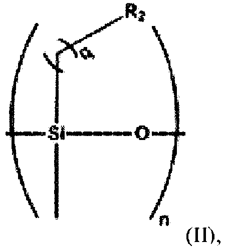 où q est compris entre 2 et 18, n est compris entre 1 et 50 et R₂ est choisi parmi le groupe constitué de composés de 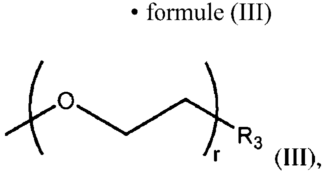 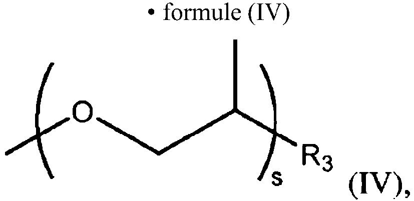 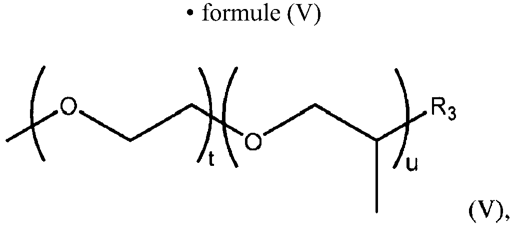 and 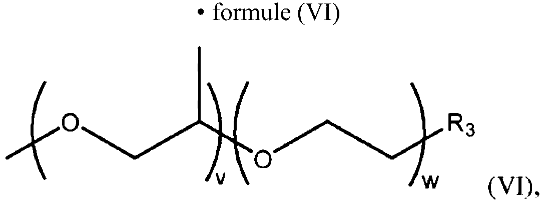 où chacun de r, s, t, u, v et w est compris entre 1 et 32 et R₃ est choisi parmi le groupe constitué de -OH, -O-CH₃ et -O-CH₂CH₃ ;
- le bloc [C] du tensioactif correspond à un bloc de formule (VII) 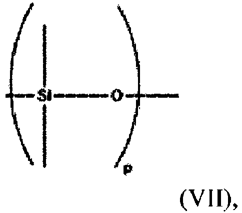
où p est compris entre 5 et 300 ;
où les groupes terminaux sont choisis parmi le groupe constitué de -H, -CH₃ et -Si(CH₃) 3 ;
où la formule générale du tensioactif est choisie parmi [A]-[B]-[C], [B]-[C]-[A], [C]-[A]-[B], [B]-[A]-[C], [A]-[C]-[B] et [C]-[B]-[A] ;
et où la composition comprend au moins 0,5 % en poids du tensioactif, rapporté au poids total de la phase huileuse continue.

4. Dispositif microfluidique comprenant au moins une chambre contenant une huile de silicone et un tensioactif, dans lequel le tensioactif est un copolymère à blocs comprenant les blocs [A], [B] et [C], dans lequel :
- le bloc [A] du tensioactif correspond à un bloc de formule (I) 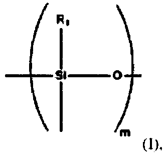 où R₁ est un alkyle en C₁-C₁₈ et m est compris entre 10 et 100 ;
- le bloc [B] du tensioactif correspond à un bloc de formule (II) 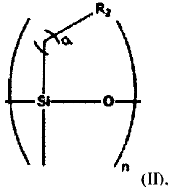 où q est compris entre 2 et 18, n est compris entre 1 et 50 et R₂ est choisi parmi le groupe constitué de composés de
• 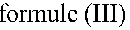 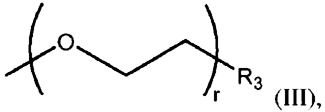 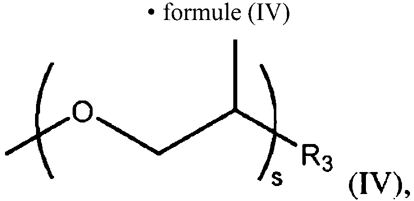 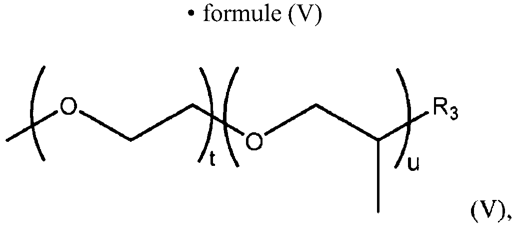 and 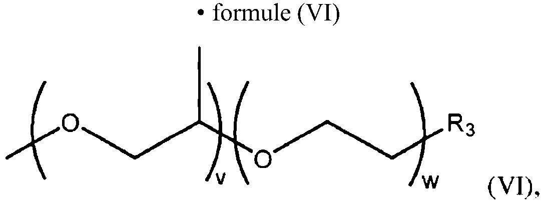 où chacun de r, s, t, u, v et w est compris entre 1 et 32 et R₃ est choisi parmi le groupe constitué de -OH, -O-CH₃ et -O-CH₂CH₃ ;
- le bloc [C] du tensioactif correspond à un bloc de formule (VII) 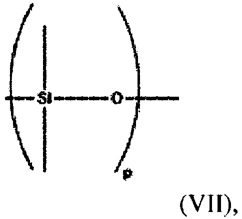
où p est compris entre 5 et 300 ;
où les groupes terminaux sont choisis parmi le groupe constitué de -H, -CH₃ et - Si(CH₃)₃ ;
où la formule générale du tensioactif est choisie parmi [A]-[B]-[C], [B]-[C]-[A], [C]-[A]-[B], [B]-[A]-[C], [A]-[C]-[B] et [C]-[B]-[A].

5. Procédé selon la revendication 1, utilisation selon la revendication 2, phase huileuse continue selon la revendication 3 et dispositif microfluidique selon la revendication 4, dans lesquels
- le bloc [A] du tensioactif correspond à un bloc de formule (I) 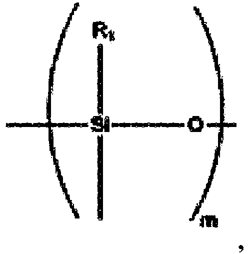 où R₁ est un alkyle en C₁₂ et m est compris entre 10 et 35 ;
- le bloc [B] du tensioactif correspond à un bloc de formule (II) 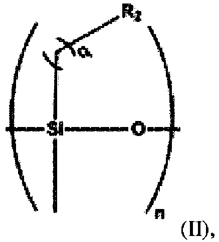 où q vaut 3, n est compris entre 1 et 15 et R₂ est un composé de formule (III) 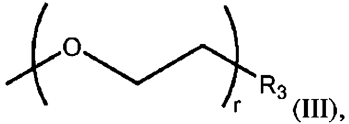 où r est compris entre 6 et 9 et R₃ est -OH ;
- le bloc [C] du tensioactif correspond à un bloc de formule (VII) 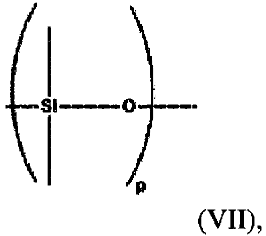
où p est compris entre 75 et 175 ;
où les groupes terminaux sont choisis parmi le groupe constitué de H, CH₃ et -Si(CH₃)₃.

6. Procédé selon la revendication 1, utilisation selon la revendication 2, phase huileuse continue selon la revendication 3 et dispositif microfluidique selon la revendication 4, dans lesquels
- le bloc [A] du tensioactif correspond à un bloc de formule (I) 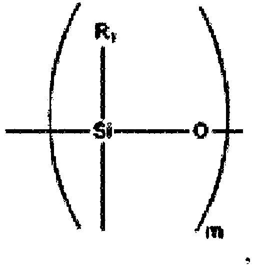 où R₁ est un alkyle en C₁₆ et m est compris entre 25 et 75 ;
- le bloc [B] du tensioactif correspond à un bloc de formule (II) 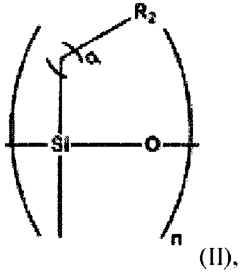 où q vaut 3, n est compris entre 1 et 5 ou entre 5 et 15 et R₂ est un composé de formule (V) 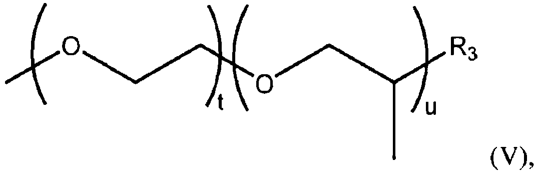 où u vaut 1, t vaut 10 et R₃ est -OH ;
- le bloc [C] du tensioactif correspond à un bloc de formule (VII) 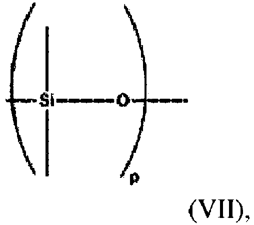
où p est compris entre 100 et 300 ;
où les groupes terminaux sont choisis parmi le groupe constitué de H, CH₃ et -Si(CH₃)₃.

7. Procédé, utilisation, phase huileuse continue, dispositif microfluidique selon l'une quelconque des revendications 1 à 6, dans lesquels la viscosité de l'huile de silicone est comprise entre 0,7 et 5,0 centistokes à 25 °C.

8. Procédé, utilisation, phase huileuse continue, dispositif microfluidique selon l'une quelconque des revendications 1 à 7, dans lesquels l'huile de silicone comprend du décaméthyltétrasiloxane.

9. Procédé, utilisation, phase huileuse continue, dispositif microfluidique selon l'une quelconque des revendications 1 à 8, dans lesquels l'huile de silicone comprend au moins 60 % en poids de décaméthyltétrasiloxane rapportés au poids total de l'huile de silicone.

10. Procédé, utilisation, phase huileuse continue, dispositif microfluidique selon l'une quelconque des revendications 1 à 9, dans lesquels l'huile de silicone comprend en outre de l'octaméthyltrisiloxane et/ou du dodécaméthylpentasiloxane.

11. Procédé, utilisation, phase huileuse continue, dispositif microfluidique selon l'une quelconque des revendications 1 à 10, dans lesquels la phase huileuse continue comprend 1 à 10 % en poids d'octaméthyltrisiloxane et/ou 1 à 10 % en poids de dodécaméthylpentasiloxane rapportés au poids total de l'huile de silicone.

12. Procédé selon la revendication 1, dans lequel ledit matériel biologique comprend des cellules ou des acides nucléiques.

13. Procédé selon la revendication 1, dans lequel l'étape de traitement du matériel biologique comprend l'amplification du matériel biologique, de préférence par PCR numérique.

14. Procédé, utilisation, phase huileuse continue, dispositif microfluidique selon l'une quelconque des revendications 1 à 13, dans lesquels le tensioactif est ABE 3642, dont le numéro CAS est 212335-52-9.
